(19) Europäisches Patentamt
European Patent Office
Office européen des brevets

(11) EP 3 903 827 A1

(12) # EUROPEAN PATENT APPLICATION
published in accordance with Art. 153(4) EPC

(43) Date of publication:
**03.11.2021 Bulletin 2021/44**

(21) Application number: **20738311.8**

(22) Date of filing: **03.01.2020**

(51) Int Cl.:
*A61K 47/55* (2017.01) *A61K 31/395* (2006.01)
*A61K 31/4545* (2006.01) *A61P 31/04* (2006.01)

(86) International application number:
**PCT/CN2020/070161**

(87) International publication number:
**WO 2020/143534 (16.07.2020 Gazette 2020/29)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.01.2019 CN 201910017484**

(71) Applicant: **Tennor Therapeutics Limited**
**Suzhou 215123 (CN)**

(72) Inventors:
- **MA, Zhenkun**
  **Suzhou, Jiangsu 215123 (CN)**
- **YUAN, Ying**
  **Suzhou, Jiangsu 215123 (CN)**
- **LIU, Yu**
  **Suzhou, Jiangsu 215123 (CN)**

(74) Representative: **Gong, Jinping**
**CocreateIP**
**Eggenfeldenerstraße 56**
**81929 München (DE)**

(54) **USE OF RIFAMYCIN-QUINOLIZIDONE COUPLING MOLECULE AND PHARMACEUTICALLY ACCEPTABLE SALT THEREOF**

(57) The present invention provides an application of a rifamycin-quinolizidone conjugate molecule shown in formula I and pharmaceutically acceptable salt thereof in preparation of a drug for treating or preventing infections caused by methicillin-resistant, quinolone-resistant, and methicillin and quinolone multidrug resistant *staphylococcus aureus.*

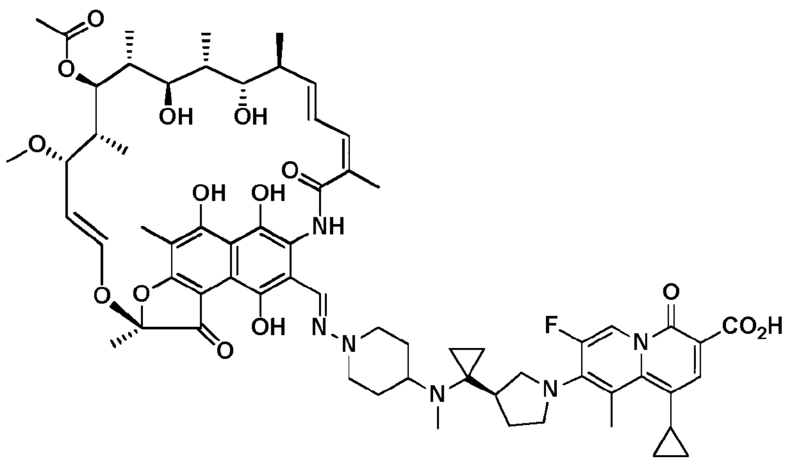

I.

The rifamycin-quinolizidone conjugate molecule and pharmaceutically acceptable salt thereof provided by the present invention can effectively treat or prevent bacterial infections and diseases caused by methicillin-resistant, quinolone-resistant, and methicillin and quinolone multidrug resistant *staphylococcus aureus,* and can reduce spontaneous resistance frequency as compared with a drug combination of rifamycin and quinolones.

EP 3 903 827 A1

**Description**

Technical Field

The present invention relates to an application of a rifamycin-quinolizinone conjugate molecule and pharmaceutically acceptable salt thereof, which belongs to the technical field of medicines.

Background

Infection caused by methicillin-resistant *staphylococcus aureus* (MRSA) is a serious clinical disease and public health problem at present. Since the first emergence of MRSA in the 1960s, the isolate rate of MRSA has increased year by year, becoming important pathogenic bacteria of hospital acquired infections and there is a trend of spreading to the community. Meanwhile, the multidrug resistant phenomenon has become more and more serious, *staphylococcus aureus* with different drug resistance to vancomycin has appeared, and most of the MRSA are also resistant to both quinolones and macrolides antibiotics. The formation of a bacterial biofilm on the human tissue and implantable medical device surface is an important condition for reducing antibiotic potency and forming drug-resistant bacteria. Therefore, treatment of infections caused by methicillin-resistant and quinolone-resistant *staphylococcus aureus,* especially biofilm-associated infections, is a current major unmet clinical need.

Summary

In view of the above defects existing in the prior art, the purpose of the present invention is to provide an application of a rifamycin-quinolizinone conjugate molecule and pharmaceutically acceptable salt thereof which are capable of effectively treating or preventing bacterial infections caused by methicillin-resistant and/or quinolone-resistant *staphylococcus aureus.*

The purpose of the present invention is achieved by means of the following technical solution:

An application of a rifamycin-quinolizidone conjugate molecule shown in formula I and pharmaceutically acceptable salt thereof in preparation of a drug for treating or preventing diseases caused by multiple-resistant *staphylococcus aureus* infections.

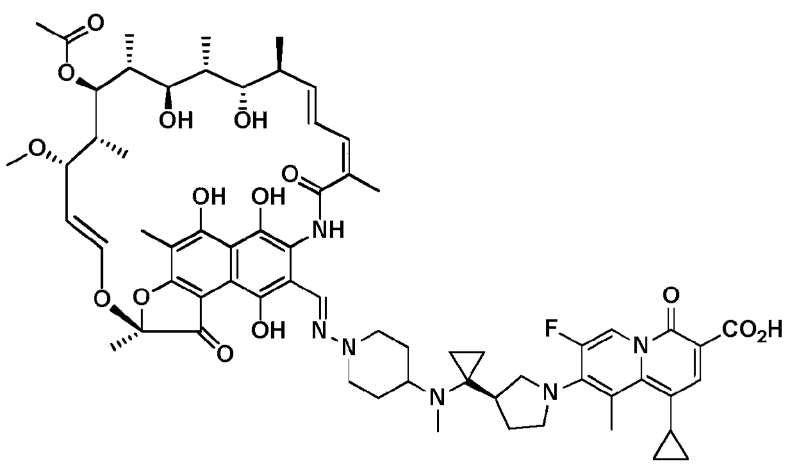

I.

In the application, preferably, the multiple-resistant *staphylococcus aureus* comprises methicillin and quinolone multidrug resistant *staphylococcus aureus,* methicillin-resistant *staphylococcus aureus* or quinolone-resistant *staphylococcus aureus.*

In the application, preferably, the infections caused by multiple-resistant *staphylococcus aureus* comprises acute bacterial infections and/or biofilm-associated bacterial infections.

In the application, preferably, the acute bacterial infections comprise skin and skin tissue infections, respiratory tract infections or bacteremia.

In the application, preferably, the biofilm-associated infections comprise one or a combination of cardiac valve infections, prosthetic joint infections and catheter related bloodstream infections.

The present invention has following prominent effects:

The rifamycin-quinolizidone conjugate molecule and pharmaceutically acceptable salt thereof provided by the present invention can effectively treat or prevent bacterial infections and diseases caused by methicillin-resistant, quinolone-resistant, and methicillin and quinolone multidrug resistant *staphylococcus aureus*, and can reduce spontaneous resistance frequency.

Description of drawings

Fig. 1a is a diagram showing an effect of 14-day treatment of a rifamycin-quinolinone conjugate molecule I on mice infected with an isogenic quinolone-resistant strain of *staphylococcus aureus;*
Fig. 1b is a diagram showing an effect of a rebound treatment scheme of a rifamycin-quinolinone conjugate molecule I on mice infected with an isogenic quinolone-resistant strain of *staphylococcus aureus*;
Fig. 2a is a diagram showing an effect of 14-day treatment of a combination of rifampicin + levofloxacin on mice infected with an isogenic quinolone-resistant strain of *staphylococcus aureus;*
Fig. 2b is a diagram showing an effect of a rebound treatment scheme of a combination of rifampicin + levofloxacin on mice infected with an isogenic quinolone-resistant strain of *staphylococcus aureus.*

Detailed Description

To understand the technical features, purpose and beneficial effects of the present invention more clearly, the technical solutions of the present invention are described in detail below, but shall not be understood as the limitation of the implementation scope of the present invention. The experimental methods described in the following embodiments are conventional methods unless otherwise specified; and the reagents and materials are commercially available unless otherwise specified.
In the following embodiments, the rifamycin-quinolinone conjugate molecule is defined as I for ease of meaning representation in the chart.

**Embodiment 1**

This embodiment provides an application of a rifamycin-quinolizidone conjugate molecule (I) and pharmaceutically acceptable salt thereof in preparation of a drug for treating or preventing infections caused by methicillin-resistant *staphylococcus aureus.*
In this embodiment, all the pharmaceutically acceptable salts (such as monosodium salt, disodium salt, potassium salt, etc.) of I may be transformed into the parent drug of I (as shown in formula I) *in vivo,* so the experimental test is based on I; I is provided by TenNor Therapeutics Ltd.

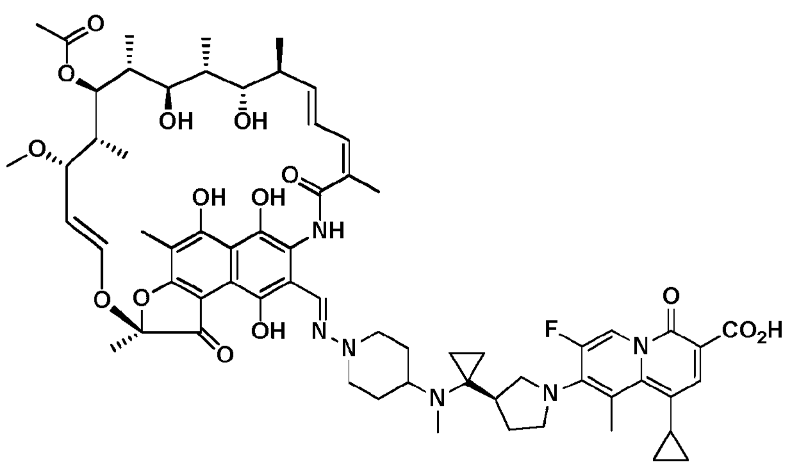

I

In the application of this embodiment, under the background of sensitivity to quinolone drugs and different degrees of drug resistance, for the analysis of sensitivity of methicillin-resistant *staphylococcus aureus* to I and the spontaneous mutation frequency resulting in drug resistance, as well as comparison with rifampicin, levofloxacin and

drug combination of rifampicin and levofloxacin, the specific methods are as follows:

**Test Drug:** rifamycin-quinolizidone conjugate molecule I was provided by TenNor Therapeutics Ltd., and was stored at -20°C. Comparator compounds are commercially available. Related information about the test compounds are shown in Table 1 below:

Table 1

| Compound | Supplier | Batch Number | Solvent |
|---|---|---|---|
| Rifamycin-quinolizidone conjugate molecule I | TenNor Therapeutics, Ltd. | C13062864-V16001M | DMSO |
| Rifampicin | Sigma | 011M1159V | Methanol |
| Oxacillin | Sigma | BCBF5635V | Water |
| Levofloxacin | Sigma | BCBF7004V | Water/sodium hydroxide (added dropwise) |

Spontaneous mutation and minimal inhibit concentration (MIC) tested by agar dilution method, the above compounds are respectively prepared into a stock solution (100X) with a concentration 100 times the highest final concentration in corresponding solvents and used on the same day. Before use, I is further diluted with DMSO; and oxacillin, rifampicin and levofloxacin are further diluted with water. In the process of performing analysis of MIC measured by agar dilution method or analysis of spontaneous mutation on the compounds, the concentrations of DMSO are not higher than 1% (v/v).

**Bacterial isolates**: *staphylococcus aureus* isolates are retrieved from the -80°C storage room of Micromyx Culture Collection, thawed, plated onto Tryptic Soy Agar + 5% sheep blood (Remel; Lenexa, KS), and then incubated aerobically at 35°C overnight. S. aureus ATCC 29213 (MRSA; MMX 0100) was included during testing only for the purpose of quality control.

**Test Media**: The isolates were tested in both the agar dilution MIC assay and the spontaneous mutation assay using Mueller-Hinton Agar (MHA; BD/BBL; Lot No. 6229829 & 4216798). Inocula of each isolate were suspended in cation-adjusted Mueller-Hinton Broth (CAMHB) (BD/BBL; Lot Nos. 5257869 & 6258541). Sodium chloride (Sigma-Aldrich; Lot No. SLBL0434V) was added to CAMHB at a final concentration of 2% when testing oxacillin. Dilutions of bacterial cell suspensions for the purpose of performing colony counts were also made in CAMHB.

**MIC measured by agar dilution method**: according to the recommendation from the Clinical and Laboratory Standards Institute (CLSI), the MIC value is measured by the agar dilution method. All test agents were serially diluted two-fold at 100X the final test concentrations (0.5 to 0.0005 μg/mL for I, 8 to 0.06 μg/mL for oxacillin, 1 to 0.001 μg/mL for rifampicin, and 64 to 0.015 μg/mL for levofloxacin). Each test agent was combined with molten MHA agar (50-55°C) at a ratio of 0.2 mL of 100X test agent to 19.8 mL agar in a sterile tube, mixed gently, and then poured into a sterile petri plate (BD Falcon). Plates were allowed to solidify at room temperature. A 0.5 McFarland standard bacterial suspension was prepared, diluted 1:10 in CAMHB, and transferred to the wells of a stainless steel replicator block before stamping onto plates containing drug, resulting in 104 CFU/spot. After allowing the inoculum to dry on the surface of the agar, the plates were inverted, incubated aerobically at 35°C for 20 - 24 hr, and inspected for growth. The MIC was defined as the lowest test agent concentration that substantially inhibited bacterial growth on the agar surface.

**Spontaneous mutation frequency determination**: *Staphylococcus aureus* MMX 3265, 3270, 3278, and 6312 were tested against I and rifampicin at 0.5, 2, and 8 μg/mL based on the agar dilution MIC results. The combination of rifampicin/levofloxacin was tested at 0.5/0.5, 2/2, and 8/8 μg/mL. Each test agent stock solution was prepared at 100X the final desired test concentration for the spontaneous mutation frequency assay agar plates.

For each of the tested isolates, 0.5 mL of either 100X I, rifampicin, or the combination of rifampicin/levofloxacin was mixed with 49.50 mL of molten MHA (49 mL for the combination compound) maintained at 50-55°C. The drug/agar mixture was dispensed into sterile 150 x 15 mm plates (BD Falcon) to a volume of 50 mL in each plate. The target inoculum for each spontaneous mutation plate was at least $10^9$ CFU. Plates were allowed to solidify at room temperature.

The inocula for the spontaneous mutation frequency assay were prepared by using sterile cotton swabs to harvest colonies from freshly grown isolates and preparing a dense cell suspension (approximately 1.70 on the turbidity meter) in 6 mL of CAMHB. The viable count of this suspension was determined by plating serial ten-fold dilutions onto MHA and taking the average of duplicate counts. For the spontaneous mutation assay, 0.25 mL of inoculum was spread onto the surface of duplicate 150 x 15 mm plates for each drug concentration tested, allowed to dry, and then the plates were inverted and incubated at 35°C for 48 hr prior to counting viable colonies. The spontaneous mutation frequency was calculated using the following equation:

Spontaneous mutation frequency = average number of colonies on selected plates/total number of cells incubated onto each plate

If there were no colonies on the antibiotic selection plates, the spontaneous mutation frequency was reported as less than or equal to the mutation frequency that would have resulted from the presence of one colony (e.g. 1/total number of cells inoculated onto the plate). In addition, if the average of the colony counts resulted in a mixed number (e.g. 16.5), the number was rounded up to the next whole number (e.g. 16.5 becomes 17).

In parallel experiments, a standard bacterium suspension with a McMahon turbidity of 0.5 is prepared as a standard inoculum for agar dilution analysis ($10^4$ CFU/point). The suspension is diluted with CAMHB in a ratio of 1:10, 2L of inoculum is transferred to each agar broth (i.e. broth used for spontaneous mutation analysis) of which the I or rifampicin concentration is 2X and 4X MIC (agar dilution method), and two plates are inoculated on each broth. In this step, the broths and drugs used in the spontaneous mutation analysis are used to confirm that the concentration of the drug stock solution can inhibit the growth of the standard inoculum. The quality of the levofloxacin stock solution is controlled by a standard inoculum and levofloxacin of 8-0.008 μg/mL, and separate agar dilution analysis is performed. This method is the best quality control scheme for the levofloxacin stock solution because most of the *staphylococcus aureus* strains in the spontaneous mutation frequency analysis are resistant to levofloxacin. The plates are incubated for 20 to 24h at 35°C and then observed。

**Confirmation of spontaneous mutation identity**: spontaneous mutants are transferred to a newly prepared agar broth for overnight incubation, and then identified by a Bruker Biotyper MALDI instrument.

**Medication result for drug-resistant bacteria**: the mutation of drug resistance of bacteria to I is evaluated by MRSA isolates resistant to levofloxacin and sensitive to rifampicin. Table 2 shows the sensitivity of 23 MRSA isolates to I, rifampicin, oxacillin and levofloxacin measured by the agar dilution method. All strains are resistant to oxacillin, indicating that they do have MRSA phenotypes.

Table 2

| Strain No. | MIC (μg/mL) | | | | |
|---|---|---|---|---|---|
| | I | Rifampicin | Oxacillin | Levofloxacin | Phenotype [1] |
| 100; ATCC 29213 | 0.015 | 0.015(0.004-0.015)[2] | 0.25(0.12-0.5)[2] | 0.25(0.06-0.5)[2] | MSSA QC strain |
| 3085 | 0.015 | 0.015 | > 8 | 0.25 | $LEV^S$ $RIF^S$ |
| 3089 | 0.015 | 0.015 | > 8 | 0.25 | Do not use this isolate[3] |
| 3090 | 0.015 | 0.008 | > 8 | 32 | $LEV^R$ $RIF^S$ |
| 3094 | 0.015 | 0.015 | > 8 | > 64 | $LEV^R$ $RIF^S$ |
| 3098 | 0.015 | 0.015 | > 8 | 0.25 | $LEV^S$ $RIF^S$ |
| **3265** | **0.015** | **0.008** | **> 8** | **64** | **$LEV^R$ $RIF^S$** |
| 3266 | 0.015 | 0.008 | > 8 | 64 | $LEV^R$ $RIF^S$ |
| **3270** | **0.015** | **0.015** | **> 8** | **4** | **$LEV^R$ $RIF^S$** |
| 3271 | 0.015 | 0.015 | > 8 | 4 | $LEV^R$ $RIF^S$ |
| 3277 | 0.015 | 0.015 | > 8 | 0.25 | $LEV^S$ $RIF^S$ |
| **3278** | **0.015** | **0.015** | **> 8** | **0.25** | **$LEV^S$ $RIF^S$** |
| **6312** | **0.015** | **0.015** | **> 8** | **16** | **$LEV^R$ $RIF^S$** |
| 6313 | 0.015 | 0.015 | > 8 | 16 | $LEV^R$ $RIF^S$ |
| 6315 | 0.015 | 0.015 | > 8 | > 64 | $LEV^R$ $RIF^S$ |
| 6498 | 0.015 | 0.015 | > 8 | 8 | $LEV^R$ $RIF^S$ |
| 6501 | 0.015 | 0.015 | > 8 | 8 | $LEV^R$ $RIF^S$ |
| 6505 | 0.015 | 0.015 | > 8 | 4 | $LEV^R$ $RIF^S$ |
| 6506 | 0.015 | 0.015 | > 8 | 0.25 | Do not use this |

(continued)

| Strain No. | MIC (μg/mL) | | | | |
|---|---|---|---|---|---|
| | I | Rifampicin | Oxacillin | Levofloxacin | Phenotype [1] |
| 7771 | 0.015 | 0.008 | > 8 | > 64 | $LEV^R$ $RIF^S$ |
| 7772 | 0.015 | 0.015 | > 8 | 0.25 | $LEV^S$ $RIF^S$ |
| 7773 | 0.015 | 0.015 | > 8 | 8 | $LEV^R$ $RIF^S$ |
| 7774 | 0.015 | 0.015 | > 8 | > 64 | $LEV^R$ $RIF^S$ |
| 7775 | 0.015 | 0.015 | > 8 | 16 | $LEV^R$ |

Note: the meanings of the superscripts in the table are as follow: represents phenotype classification based on available breakpoints, [2] represents quality control range of CLSI, [3] represents unclear endpoint; and bold represents the strain selected for spontaneous mutation assay.

I has strong inhibition activity against MRSA, and has an MIC of 0.015 μg/mL for all strains under test, which is similar to that of rifampicin. All the 23 strains are sensitive to rifampicin (if MIC $\leq$ 1 μg/mL, sensitive), but the value range of the MIC of levofloxacin is 0.25-64 μg/mL. In this embodiment, $LEV^R$ $RIF^S$ isolates 3265, 3270 and 6312 and $LEV^S$ $RIF^S$ isolates 3278 are selected as test strains for spontaneous mutation frequency analysis.

Table 3 shows the results obtained by performing spontaneous mutation frequency analysis on four MRSA isolates using the broth containing I or rifampicin (with a concentration of 0.5, 2 or 8 μg/mL). In addition, plates containing equal amount of combination of rifampicin and levofloxacin (0.5, 2 or 8 μg/mL respectively) are prepared. The inoculation size of each plate is 3.8-5.4 $\times$ $10^9$ CFU. No spontaneous mutants are recovered from the plates of I, so the spontaneous mutation frequency range thereof is less than 1.85-2.56$\times 10^{-10}$. In contrast, although it is selected that the concentration of rifampicin in the broth is 63-1000 times that of MIC, a large number of spontaneous mutants are still obtained from all strains under test.

No spontaneous mutants of the $LEV^S$ $RIF^S$ strain 3278 are screened out from the broth of the combination of rifampicin/levofloxacin, possibly because the contents of both drugs are much higher than the MIC thereof. However, except for 6312 and the plate containing rifampicin and levofloxacin of 8 μg/mL respectively (the concentration of levofloxacin is higher than MIC), all the other $LEV^R$ $RIF^S$ strains develop drug resistance to the combinations of rifampicin and levofloxacin at various concentrations. The result indicates that when rifampicin and levofloxacin are combined and the concentration of levofloxacin is higher than MIC, the generation of spontaneous mutants is inhibited, while when the concentration of levofloxacin is lower than MIC, the combination cannot inhibit the generation of drug-resistant mutants.

The quality control tests for media and drug stock solution are shown in Table 4. The results indicate that the value of MIC is already determined within the quality control range, so this experimental method is valid. It is confirmed that the concentration of the drug in agar has a strong inhibitory effect on the strains under test by aseptic growth after inoculation at the standard inoculation size on selected broths of which the concentrations of I and rifampicin are 2 times and 4 times MIC.

Table 3

| Isolate No./ Phenotype | Drug | MIC (μg/mL) agar dilution method | Drug concentration (μg/mL) in spontaneous mutation plates | Inoculation size (CFU) | Cell counts plate A | Cell counts plate B | Cell counts (mean) | Mutation frequency |
|---|---|---|---|---|---|---|---|---|
| 3278 $LEV^S$ $RIF^S$ | I | 0.015 | 0.5 | 3.80E+09 | 0 | 0 | 1 | < 2.63E-10 |
| | | | 2 | 3.80E+09 | 0 | 0 | 1 | < 2.63E-10 |
| | | | 8 | 3.80E+09 | 0 | 0 | 1 | < 2.63E-10 |
| | Rifampicin | 0.015 | 0.5 | 3.80E+09 | 49 | 44 | 47 | 1.22E-08 |
| | | | 2 | 3.80E+09 | 20 | 37 | 29 | 7.50E-09 |
| | | | 8 | 3.80E+09 | 34 | 33 | 34 | 8.82E-09 |
| | Rifampicin + levofloxacin[2] | 0.25 (LEV)[3] | 0.5 | 3.80E+09 | 0 | 0 | 1 | < 2.63E-10 |
| | | | 2 | 3.80E+09 | 0 | 0 | 1 | < 2.63E-10 |
| | | | 8 | 3.80E+09 | 0 | 0 | 1 | < 2.63E-10 |
| 3270 $LEV^R$ $RIF^S$ | I | 0.015 | 0.5 | 5.30E+09 | 0 | 0 | 1 | < 1.89E-10 |
| | | | 2 | 5.30E+09 | 0 | 0 | 1 | < 1.89E-10 |
| | | | 8 | 5.30E+09 | 0 | 0 | 1 | < 1.89E-10 |
| | Rifampicin | 0.015 | 0.5 | 5.30E+09 | 93 | 73 | 83 | 1.57E-08 |
| | | | 2 | 5.30E+09 | 44 | 53 | 49 | 9.15E-09 |
| | | | 8 | 5.30E+09 | 43 | 42 | 43 | 8.02E-09 |
| | Rifampicin + levofloxacin[2] | 4 (LEV)[3] | 0.5 | 5.30E+09 | 69 | 91 | 80 | 1.51E-08 |
| | | | 2 | 5.30E+09 | 52 | 52 | 52 | 9.81E-09 |
| | | | 8 | 5.30E+09 | 0 | 0 | 1 | < 1.89E-10 |

(continued)

| Isolate No./ Phenotype | Drug | MIC (µg/mL) agar dilution method | Drug concentration (µg/mL) in spontaneous mutation plates | Inoculation size (CFU) | Cell counts plate A | Cell counts plate B | Cell counts (mean) | Mutation frequency |
|---|---|---|---|---|---|---|---|---|
| 6312 $LEV^R$ $RIF^S$ | I | 0.015 | 0.5 | 3.90E+09 | 0 | 0 | 1 | < 2.56E-10 |
| | | | 2 | 3.90E+09 | 0 | 0 | 1 | < 2.56E-10 |
| | | | 8 | 3.90E+09 | 0 | 0 | 1 | < 2.56E-10 |
| | Rifampicin | 0.015 | 0.5 | 3.90E+09 | 34 | 29 | 32 | 8.08E-09 |
| | | | 2 | 3.90E+09 | 36 | 22 | 29 | 7.44E-09 |
| | | | 8 | 3.90E+09 | 38 | 29 | 34 | 8.59E-09 |
| | Rifampicin + levofloxacin[2] | 16 (LEV)[3] | 0.5 | 3.90E+09 | 28 | 38 | 33 | 8.46E-09 |
| | | | 2 | 3.90E+09 | 30 | 48 | 39 | 1.00E-08 |
| | | | 8 | 3.90E+09 | 0 | 0 | 1 | < 2.56E-10 |
| 3265 $LEV^R$ $RIF^S$ | I | 0.015 | 0.5 | 5.40E+09 | 0 | 0 | 1[1] | < 1.85E-10 |
| | | | 2 | 5.40E+09 | 0 | 0 | 1 | < 1.85E-10 |
| | | | 8 | 5.40E+09 | 0 | 0 | 1 | < 1.85E-10 |
| | Rifampicin | 0.008 | 0.5 | 5.40E+09 | 52 | 57 | 55 | 1.01E-08 |
| | | | 2 | 5.40E+09 | 27 | 30 | 29 | 5.28E-09 |
| | | | 8 | 5.40E+09 | 43 | 29 | 36 | 6.67E-09 |
| | Rifampicin + levofloxacin[2] | 64 (LEV)[3] | 0.5 | 5.40E+09 | 28 | 43 | 36 | 6.57E-09 |
| | | | 2 | 5.40E+09 | 43 | 22 | 33 | 6.02E-09 |
| | | | 8 | 5.40E+09 | 48 | 14 | 31 | 5.74E-09 |

Note: in the table, if no colonies are grown on spontaneous mutation plates, 1 was used to calculate the spontaneous mutation frequency. The superscript 2 represents the plates containing rifampicin and levofloxacin of 0.5, 2 or 8 μg/mL respectively, and the superscript 3 represents the MIC of levofloxacin.

Table 4

| Strain | MIC (μg/mL) measured by fluid broth microdilution method | | |
|---|---|---|---|
| | **I** | **Rifampicin** | **Levofloxacin** |
| *Staphylococcus aureus* ATCC 29213 | 0.25 | 0.008 (0.004-0.015)[1] | 0.25 (0.06-0.5)[1] |
| | **MIC (μg/mL) measured by agar dilution method** | | |
| | **I** | **Rifampicin** | **Levofloxacin** |
| | | | 0.25 (0.06-0.5)[1] |

Note: the superscript 1 in the table represents the quality control range of CLSI.

In conclusion, none of the four MRSA strains under test develops drug resistance under the condition where the concentration of I is 0.5, 2 or 8 μg/mL. The spontaneous mutation frequency of rifampicin during single drug test is high, which is in line with the expectation. The combination of rifampicin/levofloxacin can prevent drug-resistant mutations only when the concentration of levofloxacin is higher than the MIC thereof (including $LEV^S$ $RIF^S$ strains at all concentrations and $LEV^R$ $RIF^S$ strains resistant to levofloxacin of ≤ 8 μg/mL).

**Embodiment 2**

This embodiment provides an application of a rifamycin-quinolizidone conjugate molecule and pharmaceutically acceptable salt thereof in preparation of a drug for treating or preventing infections caused by methicillin-resistant *staphylococcus aureus.* The medicinal components thereof include a rifamycin-quinolizidone conjugate molecule (I) and antimicrobial agents; the dose thereof may be adjusted at random.

Meanwhile, in this embodiment, the drug preparation is tested for therapeutic effect resisting biofilm infections, i.e. *in vivo* therapeutic effect of the rifamycin-quinolizidone conjugate molecule in formula I, rifampicin, levofloxacin and gatifloxacin (used separately or jointly) in a mouse biofilm implantation model using isogenic multiple-resistant *staphylococcus aureus* strains.

**Test Drug:** rifamycin-quinolizidone conjugate molecule I was provided by TenNor Therapeutics, Ltd., and was stored at -20°C. Other test compounds and reagents are commercially available. In this embodiment, all the pharmaceutically acceptable salts (such as monosodium salt, disodium salt, potassium salt, etc.) of the rifamycin-quinolizidone conjugate molecule I may be transformed into the parent drug of I (as shown in formula I) *in vivo,* so the experimental test is based on I. In the MIC test, 0.002% polysorbate-80 (Tween) was added into the prepared solution of I. The formulation for test was prepared from 10% ethanol/5% sodium bicarbonate/6% cremaphor EL (by volume). For remaining test agents, aqueous vehicles of sterile water, 5% sodium bicarbonate or 0.9% normal saline were added to the compound shown in formula I. The rifamycin-quinolizidone conjugate molecule formulation shown in formula I was administered at 30 mg/kg. Cocktail mixtures of rifampicin and levofloxacin were prepared at 20 mg/kg + 25 mg/kg to simulate clinical $C_{max}$ exposure.

**Bacteria:** The bacteria used were biofilm forming *Staphylococcus aureus* strains. These were a wild-type parent biofilm strain CB1406 and it's isogenic fluoroquinoline-resistant derivatives CB1823 (*parC*$^{S80F}$), CB1840 (*norA*$^{up}$) and CB1857 (*parC*$^{S80F}$ + *norA*$^{up}$). Minimal inhibit concentrations (MICs) were determined by broth microdilution assays recommended by CLSI.

**Test animals**: female 5-6 week old Balb/c mice were used in this embodiment. The mice had free access to food and water during the course of this test and animal procedures were compliant with UNTHSC Institutional Animal Care and Use Committee (IACUC) guidelines.

**In vitro preparation of colonized implants:** Teflon venous catheters (14-Guage AbboCath-T#271-1000 or Braun Introcan#425 2594) were cut into 1 cm segments, sterilized by soaking in 70% ethanol for 15 minutes, dried under UV light for 20 minutes followed by aseptic placement in sterile 1.5 mL polypropylene tubes warmed to 37°C. Inocula were prepared by growing overnight broth cultures, which were diluted in 1:10 in tryptic soy broth plus 0.25% glucose (TSBG) and incubated with 250 rpm at 37°C for 1 hour. Cultures were diluted to 1.0E + $0^6$ CFU/mL in 37°C TSBG, 1 mL was added to each catheter tube, incubated at 37°C without shaking for an additional 2.5 hours, and then cooled to 18°C to slow growth until implant surgerieswere completed.

**Formation and treatments of subcutaneous implant infections**: the mice were anesthetized with isoflurane

vapors (4 L/min). Each flank was shaved, sterilized with ethanol/betadine and two 3 mm incisions made . Subcutaneous pockets were excavated, then catheter segments pre-colonized with adherent bacteria inserted into the pockets closed with surgical staples. The mice were returned to their cages and regularly monitored for adverse reactions. Treatments were initiated one week post surgery (bacterial colonization and biofilm formation) twice daily by intraperitoneal injections (IP) for 7 days,7 days treatment plus a 7 days rebound period with no treatment and 7 days treatment plus7 days rebound + 7 days re-treatment with 5 mice (total 10 implants) per treatment or sampling group. Implant-associated bacteria were monitored by sampling colonized catheter segments during the course of implant surgeries and at regular intervals for up to 28 days post infection.

**Sampling of infected implants:** infected mice were euthanized by $CO_2$ inhalation, then flanks were sterilized with 70% ethanol, and the infected catheter segments were excised into 1 mL of phosphate buffered saline pH 7.2 (1×PBS). Bacteria that adhered to the internal surfaces of explanted catheter segments were mechanically disrupted (aseptically) by repeated draws/dispenses of 200μL 1x PBS from a micropipette tip inserted into catheter lumens, followed by 5 min vortexing at high speed. Explanted samples were then snap frozen in dry ice/ethanol and stored at -80°C until CFU counts were determined.

**CFU count determination:** the catheters were rapidly thawed in a 37°C water bath, and vortexed for 2 minutes, serially diluted and spotted (8 μL) on agar plates. Viable cell counts for efficacy were determined by plating on drug free Charcoal or MHII agar plates. Survival and emergence of drug resistant bacteria were evaluated by parallel plating of diluted samples on separate MHII agar plates containing 1 μg/mL (rifampicin, levofloxacin, I) of appropriate test agents. The detection limit for all CFU determinations was less than or equal to 2.09 $log_{10}$ CFU/implant.

**Data analysis:** CFU/catheter were determined by counting colonies at a dilution that resulted in 5-50 colonies/spot. The counts were multiplied by the serial dilution factor. The resulting raw values were converted to $log_{10}$ CFU for calculation of means and standard deviations. Log reductions for treatment groups were determined by subtracting mean $log_{10}$ CFU values of drug treated mice from the mean $log_{10}$ CFU values of vehicle treated mice.

**Results:** Figs. 1a and 1b show a therapeutic effect of the rifamycin-quinolinone conjugate molecule I on mice infected with an isogenic strain of *staphylococcus aureus*; mean $log_{10}$ CFU/catheter of mice treated with vehicle and mice treated with antibiotics. Treatments were initiated twice daily by IP injection according to the following regimen: 1-1) 14 days treatment: administration for 14 consecutive days from one week post infection; 1-2) rebound treatment: treatment for 7 consecutive days from one week post infection, following a 7 day rebound period with no treatment and an additional 7 treatment days. $Log_{10}$ CFU counts were determined at the initiation of treatment (day 7), day 14 (after 7 days of administration), day 21 (after 14 days of treatment or 7 days of rebound), and day 28 (7 days of re-treatment).The detection limit was 2.09 $log_{10}$ CFU/implant.

Fig. 2a and Fig. 2b show a therapeutic effect of the combination of rifampicin + levofloxacin on mice infected with an isogenic strain of *staphylococcus aureus*; mean $log_{10}$ CFU/catheter of mice treated with vehicle and mice treated with antibiotics. Treatments were initiated twice daily by IP injection according to the following regimen: 2-1) 14 days treatment: administration for consecutive 14 days from one week post infection; 2-2) rebound treatment: treatment for consecutive 7 days from one week post infection, following a 7 day rebound period with no treatment and an additional 7 treatment days. $Log_{10}$ CFU counts were determined at the initiation of treatment (day 7), day 14 (after 7 days of administration), day 21 (after 14 days of treatment or 7 days of rebound), and day 28 (7 days of re-treatment). The detection limit was 2.09 $log_{10}$ CFU/implant.

The results of the rifamycin-quinolizidone conjugate molecule I and rifampicin + levofloxacin in treatment of *staphylococcus aureus* CB1406 (wild type) are shown in Tables 5-1 and 5-2, and Figs. 1a, 1b, 2a and 2b.

Table 5-1 14-day treatment regimen (mean $Log_{10}$ CFU/catheter)

| Treatment group | Day 7 (before treatment) | Day 21 |
|---|---|---|
| Vehicle | 6.82 | 7.20 |
| Rifamycin-quinolizidone conjugate molecule I | 6.82 | 2.20 |
| Rifampicin + levofloxacin | 6.82 | 2.10 |

Table 5-2 Rebound treatment regimen (mean $Log_{10}$ CFU/catheter)

| Treatment group | Day 7 (before treatment) | Day 14 | Day 21 | Day 28 |
|---|---|---|---|---|
| Vehicle | 6.82 | 7.29 | 7.26 | 7.18 |
| Rifamycin-quinolizi done conjugate molecule I | 6.82 | 4.68 | 2.20 | 2.10 |

(continued)

| Treatment group | Day 7 (before treatment) | Day 14 | Day 21 | Day 28 |
|---|---|---|---|---|
| Rifampicin + levofloxacin | 6.82 | 5.28 | 2.96 | 2.87 |

Administration of I twice daily by IP injection at 30mg/kg bid for 14 consecutive days resulted in a 4.6 log reduction in bacterial colony counts at the catheter site of infection for this wild-type *staphylococcus aureus* strain. The combination of rifampicin + levofloxacin at 20 + 25 mg/kg for the same treatment regimen exhibited a comparable reduction (4.7 log) in counts. No resistance development, as evidenced by growth of the recovered isolates from catheters on antibiotic containing plates, was observed to either rifampicin or I in any of the I or rifampicin + levofloxacin treated animals.

During the rebound treatment regimen, I affected 2.1, 4.6 and 4.7 $log_{10}$ CFU reductions in bacterial counts following 7 days of treatment, 7 days untreated and an additional 7 treatment days , respectively. Rifampicin + levofloxacin was slightly less efficacious than the rifamycin-quinolinone conjugate molecule I, exhibiting 1.5, 3.8 and 4.0 $log_{10}$ CFU reductions in bacterial counts during these same time periods, respectively. No resistance to rifampicin or I was observed in the I treated animals at all sampling points. In contrast, after 7 days of re-treatment, approx. 25% of the catheters from the rifampicin + levofloxacin treated animals exhibited resistance to rifampicin.

The results of the rifamycin-quinolizidone conjugate molecule I and the drug combination of rifampicin + levofloxacin in treatment of quinolone-resistant *staphylococcus aureus* CB1823 (*parC*$^{S80F}$) are shown in Tables 6-1 and 6-2, and Figs. 1a, 1b, 2a and 2b.

Table 6-1 14-day treatment regimen (mean $Log_{10}$ CFU/catheter)

| Treatment group | Day 7 | Day 21 |
|---|---|---|
| Vehicle | 7.03 | 7.20 |
| Rifamycin-quinolizidone conjugate molecule I | 7.03 | 2.70 |
| Rifampicin + levofloxacin | 7.03 | 2.80 |

Table 6-2 Reboundtreatment regimen (mean $Log_{10}$ CFU/catheter)

| Treatment group | Day 7 | Day 14 | Day 21 | Day 28 |
|---|---|---|---|---|
| Vehicle | 7.03 | 6.96 | 7.31 | 7.41 |
| Rifamycin-quinolizidone conjugate molecule I | 7.03 | 4.61 | 2.61 | 2.70 |
| Rifampicin + levofloxacin | 7.03 | 4.75 | 3.87 | 3.34 |

Administration of I at 30mg/kg bid for 14 consecutive days resulted in a 4.3 log reduction in bacterial counts at the catheter site of infection for this *staphylococcus aureus* strain CB1823 (*parC*$^{S80f}$). The combination of rifampicin + levofloxacin at 20 + 25 mg/kg for the same treatment regimen exhibited a comparable reduction (4.2 log) in counts. No resistance development, as evidenced by growth of the recovered isolates from catheters on antibiotic containing plates, was observed to either rifampicin or I in any of the I treated animals. In contrast, 25% of the rifampicin + levofloxacin treated treatment groups exhibited resistance to rifampicin.

During the rebound treatment regimen, the rifamycin-quinolinone conjugate molecule I affected 2.4, 4.4 and 4.5 $log_{10}$ CFU reductions in bacterial counts following 7 days of treatment, 7 days untreated and an additional 7 treatment days, respectively. re-. Rifampicin + levofloxacin was slightly less efficacious than the rifamycin-quinolinone conjugate molecule I, exhibiting 2.2, 3.1 and 3.6 $log_{10}$ CFU reductions in bacterial counts during these same time periods, respectively. Resistance to rifampicin was observed for the rifampicin + levofloxacin treated animals. The percent of resistant catheters after 7 days treatment, 7 days untreated and 7 days re-treatment were 0%, 30% and 30%, respectively.

The results of the rifamycin-quinolizidone conjugate molecule I and the combination of rifampicin + levofloxacin in treatment of quinolone-resistant *staphylococcus aureus* CB1840 (*norA*$^{up}$) caused by efflux pump activation are shown in Tables 7-1 and 7-2, and Figs. 1a, 1b, 2a and 2b.

Table 7-1 14-day treatment regimen (mean $Log_{10}$ CFU/catheter)

| Treatment group | Day 7 | Day 21 |
|---|---|---|
| Vehicle | 7.01 | 7.20 |
| Rifamycin-quinolizidone conjugate molecule I | 7.01 | 2.10 |
| Rifampicin + levofloxacin | 7.01 | 2.10 |

Table 7-2 Rebound treatment regimen (mean $Log_{10}$ CFU/catheter)

| Treatment group | Day 7 | Day 14 | Day 21 | Day 28 |
|---|---|---|---|---|
| Vehicle | 7.01 | 7.27 | 7.25 | 7.17 |
| Rifamycin-quinolizidone conjugate molecule I | 7.01 | 4.61 | 2.10 | 2.10 |
| Rifampicin + levofloxacin | 7.01 | 5.10 | 2.50 | 2.30 |

Administration of the rifamycin-quinazinone conjugate molecule I at 30mg/kg bid for 14 consecutive days resulted in a 4.9 $log_{10}$ CFU reduction in bacterial counts at the catheter site of infection for the *staphylococcus aureus* strain CB1840 (*norA*$^{up}$). The combination of rifampicin + levofloxacin at 20 + 25 mg/kg for the same treatment regimen exhibited a comparable reduction (4.9 $log_{10}$ CFU) in counts. No resistance development, as evidenced by growth of the recovered isolates from catheters on antibiotic containing plates, was observed to either rifampicin or rifampicin-quinazinone conjugate molecule I in any of the rifampicin-quinazinone conjugate molecule I or rifampicin + levofloxacin treated animals.

During the rebound treatment regimen, the rifamycin-quinolinone conjugate molecule I affected 2.4, 4.9 and 4.9 $log_{10}$ CFU reductions in bacterial counts following 7 days of treatment, 7 days untreated and an additional 7 treatment days, respectively. Rifampicin + levofloxacin was slightly less efficacious than the rifamycin-quinolinone conjugate molecule I, exhibiting 2.4, 4.9 and 4.9 $log_{10}$ CFU reductions in bacterial counts during these same time periods, respectively. No resistance to rifampicin or rifamycin-quinolizidone conjugate molecule I was observed for the the rifamycin-quinolizidone conjugate molecule I treated animals at all sampling points. In contrast, after 7 days of re-treatment, approx. 11% of the catheters from the rifampicin + levofloxacin treated animals exhibited resistance to rifampicin.

The results of the rifamycin-quinolizidone conjugate molecule I and the combination of rifampicin + levofloxacin in treatment of *staphylococcus aureus* CB1857 (*parC*$^{S80F}$ + *norA*$^{up}$) containing double quinolone-resistant mechanism are shown in Tables 8-1 and 8-2, and Figs. 1a, 1b, 2a and 2b.

Table 8-1 14-day treatment regimen (mean $Log_{10}$ CFU/catheter)

| Treatment group | Day 7 | Day 21 |
|---|---|---|
| Vehicle | 6.81 | 7.20 |
| Rifamycin-quinolizidone conjugate molecule I | 6.81 | 2.30 |
| Rifampicin + levofloxacin | 6.81 | 2.50 |

Table 8-2 Rebound treatment regimen (mean $Log_{10}$ CFU/catheter)

| Treatment group | Day 7 | Day 14 | Day 21 | Day 28 |
|---|---|---|---|---|
| Vehicle | 6.81 | 7.28 | 7.10 | 7.20 |
| Rifamycin-quinolizidone conjugate molecule I | 6.81 | 4.26 | 2.40 | 2.45 |
| Rifampicin + levofloxacin | 6.81 | 4.94 | 3.10 | 2.70 |

Administration of the rifamycin-quinazinone conjugate molecule I at 30mg/kg bid for 14 consecutive days resulted in a 4.5 $log_{10}$ CFU reduction in bacterial counts at the catheter site of infection for the *staphylococcus aureus* strain CB1857 (*parC*$^{S80F}$ + *norA*$^{up}$). The combination of rifampicin + levofloxacin at 20 + 25 mg/kg for the same treatment regimen exhibited a comparable reduction (4.3 $log_{10}$ CFU) in counts. No resistance development, as evidenced by growth of the recovered isolates from catheters on antibiotic containing plates, was observed to either rifampicin or

rifamycin-quinolinone conjugate molecule I in any of the rifamycin-quinolinone conjugate molecule I treated animals. In contrast, approx. 10% of the rifampicin + levofloxacin treated mice exhibited resistance to rifampicin.

During the rebound treatment regimen, the rifamycin-quinolinone conjugate molecule I affected 2.5, 4.4 and 4.4 $log_{10}$ CFU reductions in bacterial counts following 7 days of treatment, 7 days untreated and an additional 7 treatment days, respectively. Rifampicin + levofloxacin was slightly less efficacious than the rifamycin-quinolinone conjugate molecule I, exhibiting 1.8, 3.7 and 4.1 $log_{10}$ CFU reductions in bacterial counts during these same time periods, respectively. Resistance to rifampicin was observed for rifampicin + levofloxacin treated animals during the sampling time points. The percent of resistant catheters after 7 days treatment, 7 days untreated and 7 days re-treatment were 22%, 20% and 22%, respectively. In addition, 11 % of the catheters from the rifampicin + levofloxacin treated animals were resistant to rifampicin + levofloxacin following the 7 day re-treatment period.

In this embodiment, a subcutaneous implant infection and treatment model was established in mice for evaluating the *in vivo* efficacy of antibacterial agents against device related bacterial infections in peripheral tissues. The bacteria used were biofilm forming isogenic *S. aureus* strains s. These were a a wild-type parent biofilm strain CB1406, and its isogenic fluoroquinolone-resistant derivatives CB1823 (*parC*$^{S80F}$), CB1840 (*norA*$^{up}$) and CB1857 (*parC*$^{S80F}$ + *norA*$^{up}$).

All four strains exhibited very similar *in vivo* growth kinetics in mice, with Teflon implant associated mean values of 6.8 - 7.3 $log_{10}$ CFU/implant between days 7 and 28 post infection.

The rifamycin-quinazinone conjugate molecule I (30 mg/kg) and the combination of rifampicin + levofloxacin (20 + 25 mg/kg or 45 mg/kg total dose) demonstrated equivalent efficacy, as measured by $log_{10}$ CFU reduction in catheter colony counts, against each of the organisms tested when administered for 14 days bid. The range of $log_{10}$ CFU reduction for both treatments was 4.3 - 4.9. A slightly higher percentage of emergence of rifampicin resistance was observed for the rifampicin + levofloxacin treated animals for those infections where the organism contained a *parC*$^{S80F}$ mutation (CB1823 and CB1857) .

The rifamycin-quinazinone conjugate molecule I exhibited greater efficacy than the cocktail for the rebound dosing regimen. Mean $Log_{10}$ CFU reductions following 7 days of treatment, 7 days untreated (rebound) and 7 days of re-treatment were 2.4, 4.6 and 4.6 for rifamycin-quinazinone conjugate molecule I and 1.9, 3.8 and 4.1 for rifampicin + levofloxacin, while all biological samples were resistant to rifampicin after 7 days re-treatment when rifampicin + levofloxacin was administered.

The efficacy of the rifamycin-quinolizidone conjugate molecule I was demonstrated in the chronic biofilm infection model involving strains expressing individual or a combination of fluoroquinolone resistant phenotypes. The efficacy of the rifamycin-quinolizidone conjugate molecule I was equivalent to or slightly greater than that observed for the combination of rifampicin + levofloxacin against each of the *S. aureus* isolates tested. The rifamycin-quinolizidone conjugate molecule I achieved this at a lower total daily dose than the combination treatment. In general, less emergence of rifampicin resistance was observed in the rifamycin-quinolizidone conjugate molecule I treated mice, and no resistance to the rifamycin-quinolizidone conjugate molecule I itself was evident during the course of treatment.

Therefore, the rifamycin-quinolizidone conjugate molecule and pharmaceutically acceptable salt thereof provided by the present invention can effectively treat or prevent bacterial infections and diseases caused by methicillin-resistant, quinolone-resistant, and methicillin and quinolone multidrug resistant *staphylococcus aureus*, and can reduce spontaneous resistance frequency as compared with a drug combination of rifamycin and quinolones. and 4X MIC (agar dilution method), and two plates are inoculated on each broth. In this step, the broths and drugs used in the spontaneous mutation analysis are used to confirm that the concentration of the drug stock solution can inhibit the growth of the standard inoculum. The quality of the levofloxacin stock solution is controlled by a standard inoculum and levofloxacin of 8-0.008 μg/mL, and separate agar dilution analysis is performed. This method is the best quality control scheme for the levofloxacin stock solution because most of the *staphylococcus aureus* strains in the spontaneous mutation frequency analysis are resistant to levofloxacin. The plates are incubated for 20 to 24h at 35°C and then observed。

**Confirmation of spontaneous mutation identity**: spontaneous mutants are transferred to a newly prepared agar broth for overnight incubation, and then identified by a Bruker Biotyper MALDI instrument.

**Medication result for drug-resistant bacteria**: the mutation of drug resistance of bacteria to I is evaluated by MRSA isolates resistant to levofloxacin and sensitive to rifampicin. Table 2 shows the sensitivity of 23 MRSA isolates to I, rifampicin, oxacillin and levofloxacin measured by the agar dilution method. All strains are resistant to oxacillin, indicating that they do have MRSA phenotypes.

Table 2

| Strain No. | MIC ($\mu$g/mL) | | | | |
|---|---|---|---|---|---|
| | I | Rifampicin | Oxacillin | Levofloxacin | Phenotype [1] |
| 100; ATCC 29213 | 0.015 | 0.015(0.004-0 . 015 )[2] | 0.25 (0.12-0.5)[2] | 0.25 (0.06-0.5)[2] | MSSA QC strain |
| 3085 | 0.015 | 0.015 | > 8 | 0.25 | $LEV^S$ $RIF^S$ |
| 3089 | 0.015 | 0.015 | >8 | 0.25 | Do not use this isolate[3] |
| 3090 | 0.015 | 0.008 | >8 | 32 | $LEV^R$ $RIF^S$ |
| 3094 | 0.015 | 0.015 | >8 | > 64 | $LEV^R$ $RIF^S$ |
| 3098 | 0.015 | 0.015 | >8 | 0.25 | $LEV^S$ $RIF^S$ |
| **3265** | **0.015** | **0.008** | **>8** | **64** | **$LEV^R$ $RIF^S$** |
| 3266 | 0.015 | 0.008 | >8 | 64 | $LEV^R$ $RIF^S$ |
| **3270** | **0.015** | **0.015** | **>8** | **4** | **$LEV^R$ $RIF^S$** |
| 3271 | 0.015 | 0.015 | > 8 | 4 | $LEV^R$ $RIF^S$ |
| 3277 | 0.015 | 0.015 | > 8 | 0.25 | $LEV^S$ $RIF^S$ |
| **3278** | **0.015** | **0.015** | **> 8** | **0.25** | **$LEV^S$ $RIF^S$** |
| **6312** | **0.015** | **0.015** | **> 8** | **16** | **$LEV^R$ $RIF^S$** |
| 6313 | 0.015 | 0.015 | > 8 | 16 | $LEV^R$ $RIF^S$ |
| 6315 | 0.015 | 0.015 | > 8 | > 64 | $LEV^R$ $RIF^S$ |
| 6498 | 0.015 | 0.015 | > 8 | 8 | $LEV^R$ $RIF^S$ |
| 6501 | 0.015 | 0.015 | > 8 | 8 | $LEV^R$ $RIF^S$ |
| 6505 | 0.015 | 0.015 | > 8 | 4 | $LEV^R$ $RIF^S$ |
| 6506 | 0.015 | 0.015 | > 8 | 0.25 | Do not use this isolate[3] |
| 7771 | 0.015 | 0.008 | > 8 | > 64 | $LEV^R$ $RIF^S$ |
| 7772 | 0.015 | 0.015 | > 8 | 0.25 | $LEV^S$ $RIF^S$ |
| 7773 | 0.015 | 0.015 | > 8 | 8 | $LEV^R$ $RIF^S$ |
| 7774 | 0.015 | 0.015 | > 8 | > 64 | $LEV^R$ $RIF^S$ |
| 7775 | 0.015 | 0.015 | > 8 | 16 | $LEV^R$ |

Note: the meanings of the superscripts in the table are as follow: [1] represents phenotype classification based on available breakpoints, [2] represents quality control range of CLSI, [3] represents unclear endpoint; and bold represents the strain selected for spontaneous mutation assay.

I has strong inhibition activity against MRSA, and has an MIC of 0.015 $\mu$g/mL for all strains under test, which is similar to that of rifampicin. All the 23 strains are sensitive to rifampicin (if MIC $\leq$ 1 $\mu$g/mL, sensitive), but the value range of the MIC of levofloxacin is 0.25-64 $\mu$g/mL. In this embodiment, $LEV^R$ $RIF^S$ isolates 3265, 3270 and 6312 and $LEV^S$ $RIF^S$ isolates 3278 are selected as test strains for spontaneous mutation frequency analysis.

Table 3 shows the results obtained by performing spontaneous mutation frequency analysis on four MRSA isolates using the broth containing I or rifampicin (with a concentration of 0.5, 2 or 8 $\mu$g/mL). In addition, plates containing equal amount of combination of rifampicin and levofloxacin (0.5, 2 or 8 $\mu$g/mL respectively) are prepared. The inoculation size of each plate is 3.8-5.4 $\times$ $10^9$ CFU. No spontaneous mutants are recovered from the plates of I, so the spontaneous mutation frequency range thereof is less than 1.85-2.56$\times 10^{-10}$. In contrast, although it is selected that the concentration of rifampicin in the broth is 63-1000 times that of MIC, a large number of spontaneous mutants are still obtained from all strains under test.

No spontaneous mutants of the $LEV^S$ $RIF^S$ strain 3278 are screened out from the broth of the combination of

rifampicin/levofloxacin, possibly because the contents of both drugs are

## Claims

1. An application of a rifamycin-quinolizidone conjugate molecule shown in formula I and pharmaceutically acceptable salt thereof in preparation of a drug for treating or preventing infections caused by multiple-resistant *staphylococcus aureus.*

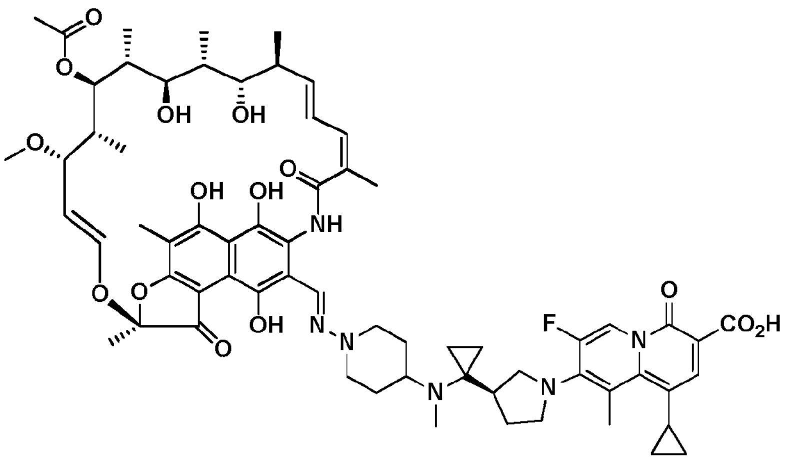

I.

2. The application according to claim 1, **characterized in that** the multiple-resistant staphylococcus aureus comprises methicillin and quinolone multidrug resistant *staphylococcus aureus*, methicillin-resistant *staphylococcus aureus* or quinolone-resistant *staphylococcus aureus.*

3. The application according to claim 1 or 2, **characterized in that** the infections caused by multiple-resistant *staphylococcus aureus* comprises acute bacterial infections and/or biofilm-associated bacterial infections.

4. The application according to claim 3, **characterized in that** the acute bacterial infections comprises skin and skin tissue infections, respiratory tract infections or bacteremia.

5. The application according to claim 3, **characterized in that** the biofilm-associated infections comprises cardiac valve infections, prosthetic joint infections and catheter related bloodstream infections.
The present invention provides an application of a rifamycin-quinolizidone conjugate molecule shown in formula I and pharmaceutically acceptable salt thereof in preparation of a drug for treating or preventing infections caused by methicillin-resistant, quinolone-resistant, and methicillin and quinolone multidrug resistant *staphylococcus aureus.*

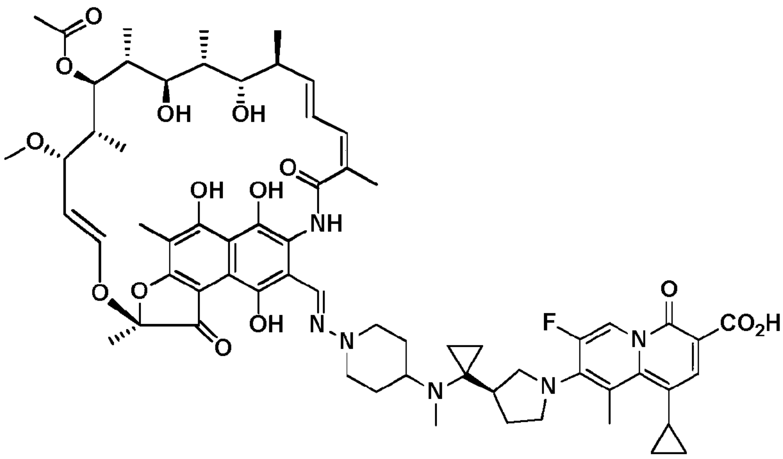

I.

The rifamycin-quinolizidone conjugate molecule and pharmaceutically acceptable salt thereof provided by the present invention can effectively treat or prevent bacterial infections and diseases caused by methicillin-resistant, quinolone-resistant, and methicillin and quinolone multidrug resistant *staphylococcus aureus,* and can reduce spontaneous resistance frequency as compared with a drug combination of rifamycin and quinolones.

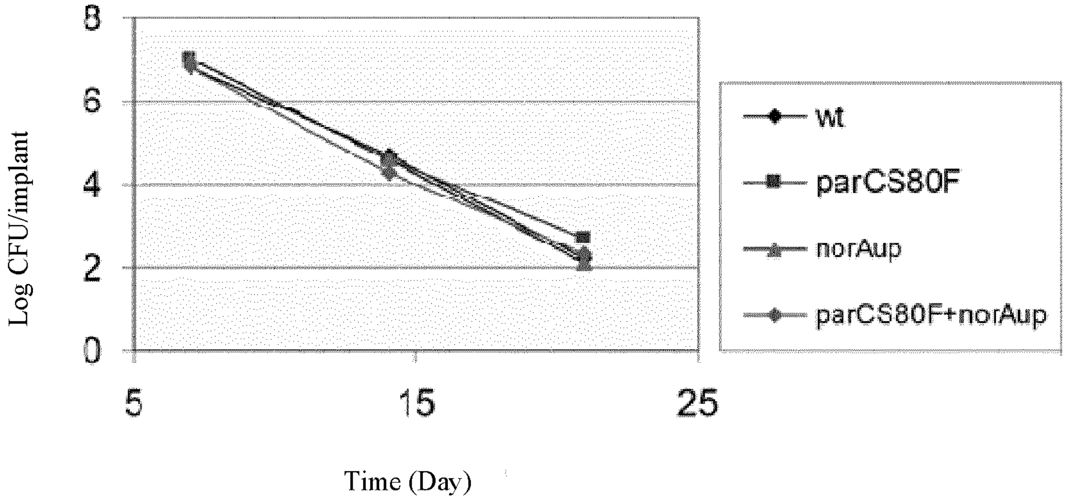


Fig. 1a

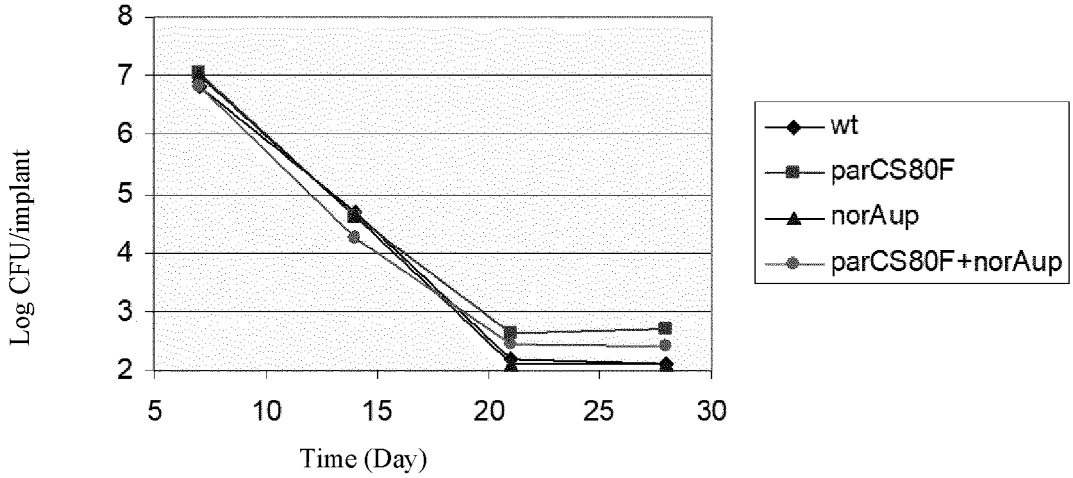


Fig. 1b

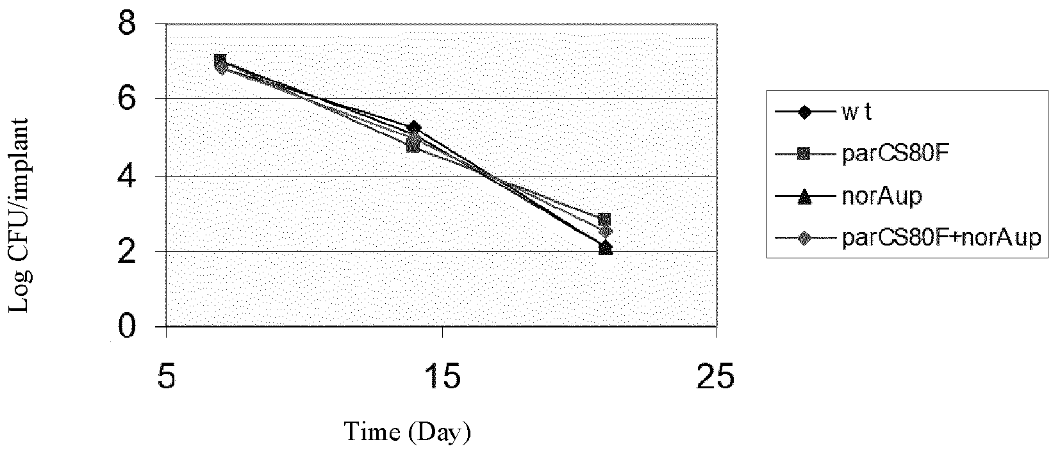


Fig. 2a

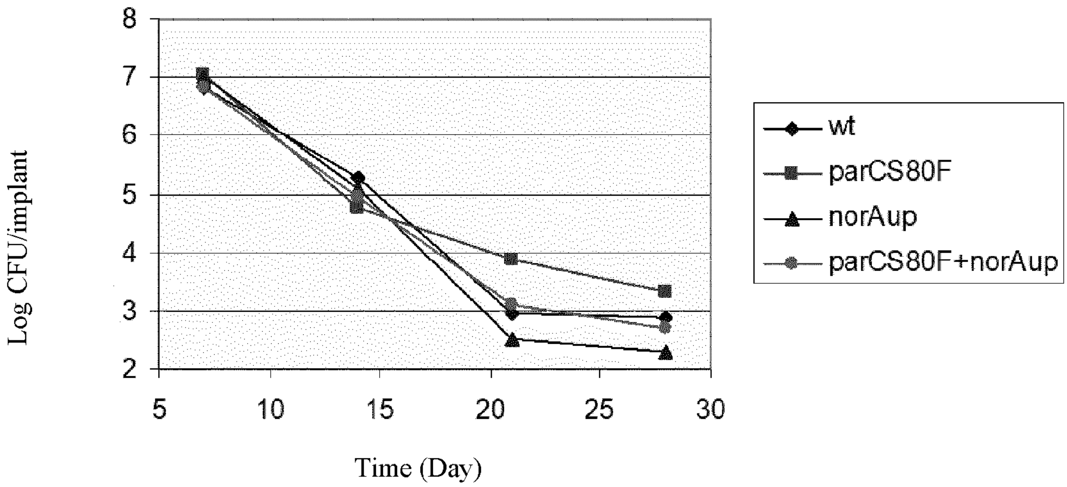
Log CFU/implant
8
7
6
5
4
3
2
5
10
15
20
25
30
Time (Day)
wt
parCS80F
norAup
parCS80F+norAup

Fig. 2b

## INTERNATIONAL SEARCH REPORT

International application No.
**PCT/CN2020/070161**

**A. CLASSIFICATION OF SUBJECT MATTER**

A61K 47/55(2017.01)i； A61K 31/395(2006.01)i； A61K 31/4545(2006.01)i； A61P 31/04(2006.01)i

According to International Patent Classification (IPC) or to both national classification and IPC

**B. FIELDS SEARCHED**

Minimum documentation searched (classification system followed by classification symbols)

A61K; A61P

Documentation searched other than minimum documentation to the extent that such documents are included in the fields searched

Electronic data base consulted during the international search (name of data base and, where practicable, search terms used)

CNABS; CNTXT; DWPI; SIPOABS; WOTXT; USTXT; EPTXT; STN; CNKI; CJFD; 万方; 读秀; ISI Web of Science: 丹诺, 马振坤, 袁鹰, 刘宇, 利福霉素, 喹嗪酮, 金黄色葡萄球菌, 耐药, 甲氧西林, 美替西林, 喹诺酮, structural formula search based on formula (I), 922717-97-3, TenNor, Ma Zhenkun, Yuan Ying, Liu Yu, rifamycin, quinazinone, staphylococcus aureus, staphylococci, resistant, methicillin, meticillin, MRSA, quinolone, QRSA, QMRSA

**C. DOCUMENTS CONSIDERED TO BE RELEVANT**

| Category* | Citation of document, with indication, where appropriate, of the relevant passages | Relevant to claim No. |
|---|---|---|
| PX | CN 109529045 A (TENNOR THERAPEUTICS (SUZHOU) LTD.) 29 March 2019 (2019-03-29)<br>claims 1-5 | 1-5 |
| PX | CN 109464673 A (TENNOR THERAPEUTICS (SUZHOU) LTD.) 15 March 2019 (2019-03-15)<br>claims 1, 4-6, description, embodiments 5, 7, 8, 10 | 1-5 |
| X | MA, Zhenkun et al. "Development of a Dual-Acting Antibacterial Agent (TNP-2092) for the Treatment of Persistent Bacterial Infections"<br>*Journal of Medicinal Chemistry,* Vol. vol. 59, 23 June 2016 (2016-06-23),<br>pages 6645-6657, tables 2, 3, page 6651, left-hand column, paragraph 1 to page 6653, left-hand column, paragraph 1 | 1-5 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **02 March 2020** | **23 March 2020** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/070161**

**C. DOCUMENTS CONSIDERED TO BE RELEVANT**

| Category* | Citation of document, with indication, where appropriate, of the relevant passages | Relevant to claim No. |
|---|---|---|
| X | ROBERTSON, Gregory T. et al. "In Vitro Evaluation of CBR-2092, a Novel Rifamycin-Quinolone Hybrid Antibiotic: Microbiology Profiling Studies with Staphylococci and Streptococci" *Antimicrobial Agents and Chemotherapy,* Vol. 52, No. 7, 31 July 2008 (2008-07-31), pages 2324-2334, tables 2, 3, page 2326, right-hand column, paragraph 1, page 2328, left-hand column, paragraph 2, page 2332, right-hand column, paragraph 3 | 1-5 |
| X | ROBERTSON, Gregory T. et al. "In Vitro Evaluation of CBR-2092, a Novel Rifamycin-Quinolone Hybrid Antibiotic: Studies of the Mode of Action in Staphylococcus aureus" *Antimicrobial Agents and Chemotherapy,* Vol. 52, No. 7, 31 July 2008 (2008-07-31), pages 2313-2323, abstract, tables 2, 3 | 1-5 |
| X | CN 101031572 A (CUMBRE PHARMACEUTICALS INC) 05 September 2007 (2007-09-05) description, paragraphs [0013], [0014], [0034]-[0036], table 1, embodiment 1 | 1-5 |

Form PCT/ISA/210 (second sheet) (January 2015)

**INTERNATIONAL SEARCH REPORT**
**Information on patent family members**

International application No.
**PCT/CN2020/070161**

| Patent document cited in search report | Publication date (day/month/year) | Patent family member(s) | Publication date (day/month/year) |
|---|---|---|---|
| CN 109529045 A | 29 March 2019 | None | |
| CN 109464673 A | 15 March 2019 | None | |
| CN 101031572 A | 05 September 2007 | US 2006019985 A1 | 26 January 2006 |
| | | WO 2006012470 A1 | 02 February 2006 |
| | | CN 101031572 B | 08 December 2010 |
| | | DE 602005013671 D1 | 14 May 2009 |
| | | EP 1768987 A1 | 04 April 2007 |
| | | JP 2008507549 A | 13 March 2008 |
| | | EP 1781670 A1 | 09 May 2007 |
| | | CA 2574301 C | 10 September 2013 |
| | | WO 2006012443 A1 | 02 February 2006 |
| | | JP 5153329 B2 | 27 February 2013 |
| | | AU 2005267054 B2 | 23 June 2011 |
| | | EP 1768987 B1 | 01 April 2009 |
| | | JP 2008507548 A | 13 March 2008 |
| | | CN 101065385 A | 31 October 2007 |
| | | US 7226931 B2 | 05 June 2007 |
| | | US 7229996 B2 | 12 June 2007 |
| | | JP 5236944 B2 | 17 July 2013 |
| | | US 2006019986 A1 | 26 January 2006 |
| | | NZ 565002 A | 28 August 2009 |
| | | AU 2005267054 A1 | 24 July 2008 |

Form PCT/ISA/210 (patent family annex) (January 2015)